# FASCICULE DE BREVET EUROPEEN

(11) **EP 3 515 386 B1**
(45) Date de publication et mention de la délivrance du brevet: **03.03.2021**
(21) Numéro de dépôt: 17783915.6
(22) Date de dépôt: 19.09.2017
(51) Int. Cl.: A61F 13/00, A61F 13/02

(54) **PANSEMENT ABSORBANT COMPRENANT UN NON-TISSE SUPERABSORBANT HYDRODELITABLE**
SAUGFÄHIGER WUNDVERBAND MIT EINEM IN WASSER AUFLÖSBAREM, SUPERABSORBIERENDEM VLIESSTOFF
ABSORBENT DRESSING COMPRISING A WATER-DISINTEGRATABLE SUPERABSORBENT NONWOVEN

(30) Priorité: 20.09.2016 FR 1658789
(43) Date de publication de la demande: 31.07.2019
(73) Titulaire: Urgo Recherche Innovation et Développement, 21300 Chenove (FR)
(72) Inventeur: PERNOT, Jean-Marc, 21000 Dijon (FR)
(74) Mandataire: Plasseraud IP
(86) Numéro de dépôt international: PCT/FR2017/052503
(87) Numéro de publication internationale: WO 2018/055278

(56) Documents cités:
- WO-A1-2012/140377
- WO-A1-2013/053410
- WO-A2-02/17982
- US-A1- 2012 036 733

## Description

La présente invention se rapporte à un pansement comprenant un support de protection imperméable et respirant, un matériau absorbant et un non-tissé superabsorbant hydrodélitable constitué de deux voiles hydrosolubles entre lesquels sont incorporées des particules ou granules de polymères super-absorbants, ainsi qu'à son utilisation pour le soin des plaies, notamment des plaies exsudatives ou chroniques, comme les ulcères, les escarres, ou des plaies aiguës comme les brûlures.

Les pansements absorbants utilisés pour le traitement des plaies sont généralement constitués de l'assemblage d'un matériau absorbant type mousse et d'un support de protection imperméable et respirant. Ces pansements peuvent comprendre une bordure adhésive qui permet de les fixer sur la peau saine qui entoure la plaie. Ils peuvent également comprendre une couche d'interface positionnée sur la mousse absorbante, pour éviter tout contact direct entre la mousse et la plaie.

La réalisation de pansements absorbants doit remplir un cahier des charges complexe et concilier des caractéristiques contradictoires. En particulier, le pansement doit présenter une bonne respirabilité tout en évitant les risques de fuites et de macération, être imperméable aux liquides et aux bactéries tout en étant respirant (c'est-à-dire perméable à la vapeur d'eau), garder sa cohésion quand on le retire, et être facile à fabriquer. Le pansement doit également être facile à poser et rester en place au cours du temps le plus longtemps possible sans altérer la peau périlésionnelle, présenter une capacité d'absorption des exsudats élevée, et ne pas altérer la cicatrisation de la plaie lors de son retrait. Le pansement doit enfin être compatible avec l'utilisation complémentaire d'un système de contention.

Ainsi, le choix des matériaux qui constituent le pansement, l'agencement desdits matériaux les uns par rapport aux autres, et les moyens d'assemblage de ces derniers sont très complexes pour obtenir toutes ces propriétés à la fois.

De nombreux documents ont proposé des solutions pour la fabrication de pansements permettant d'associer les différents matériaux nécessaires à l'obtention des propriétés souhaitées, permettant de garantir la cohésion des dispositifs tout en étant suffisamment simples à mettre en œuvre.

A cet effet, les demandes WO2012/140377 et WO2012/140378 ont proposé un nouveau moyen de fixer un matériau absorbant à un support qui permet d'obtenir un niveau de cohésion des différents matériaux assemblés suffisant quand le pansement est sec mais aussi quand il est chargé des exsudats de la plaie. Les pansements décrits dans la demande WO2012/140377 comprennent un matériau absorbant de type mousse absorbante, un support de protection imperméable aux fluides et perméable à la vapeur d'eau, ledit support étant fixé indirectement audit matériau absorbant. Ces deux matériaux fixés entre eux enferment un matériau superabsorbant composé de deux voiles hydrophiles à base de cellulose et d'un cœur absorbant comprenant des polymères superabsorbants. Les pansements décrits dans la demande WO2012/140378 comprennent quant à eux un matériau absorbant de type non tissé, un support de protection imperméable aux fluides et perméable à la vapeur d'eau, ledit support étant fixé indirectement audit matériau absorbant. Ces deux matériaux fixés entre eux enferment un matériau superabsorbant composé de deux voiles hydrophiles à base de cellulose et d'un cœur absorbant comprenant des polymères superabsorbants.

Ces dispositifs multicouches proposés par l'art antérieur sont des pansements présentant des propriétés d'absorption et de cohésion souhaitées. Toutefois, les structures complexes ainsi formées présentent parfois une trop grande rigidité qui peut se traduire par un certain inconfort pour l'utilisateur, ou une mauvaise adhérence faute d'épouser correctement la morphologie du patient. En outre, au cours de leur utilisation, ces pansements sont susceptibles de générer des points de compression, notamment au niveau des pourtours de la plaie, et de ce que l'on appelle la peau péri-lesionnelle. Cela est dû au fait que les exsudats absorbés par ledit pansement se trouvent emprisonnés, notamment par le matériau superabsorbant, lequel gonfle sans se déformer ni se décomposer. Il en résulte que le pansement devient alors très inconfortable au cours de son utilisation, en particulier sur des plaies très exsudatives.

On demeure ainsi à la recherche de pansements présentant les propriétés usuellement recherchées (une bonne respirabilité tout en évitant les risques de fuites et de macération, imperméables aux liquides et aux bactéries, respirants), lesquels présentent en outre une bonne cohésion lors de leur retrait, sont faciles à fabriquer et sont suffisamment flexibles pour épouser la morphologie du patient tout en conservant des propriétés de déformation une fois les exsudats de la plaie absorbés et en particulier ne formant pas de points de compression du fait de l'absorption desdits exsudats.

La présente invention propose un pansement permettant de répondre à l'ensemble de ces problématiques.

En particulier, l'invention a pour objet, selon un premier aspect, un pansement comprenant :
- un matériau absorbant (2),
- un support de protection imperméable aux fluides et perméable à la vapeur d'eau (4), ledit support de protection étant fixé au matériau absorbant (2), ledit pansement comprenant en outre :
- un non-tissé superabsorbant hydrodélitable (6) placé entre le matériau absorbant (2) et le support de protection (4), ledit non-tissé superabsorbant hydrodélitable (6) étant constitué de deux voiles hydrosolubles entre lesquels sont incorporées des particules ou granules de polymères super-absorbants.

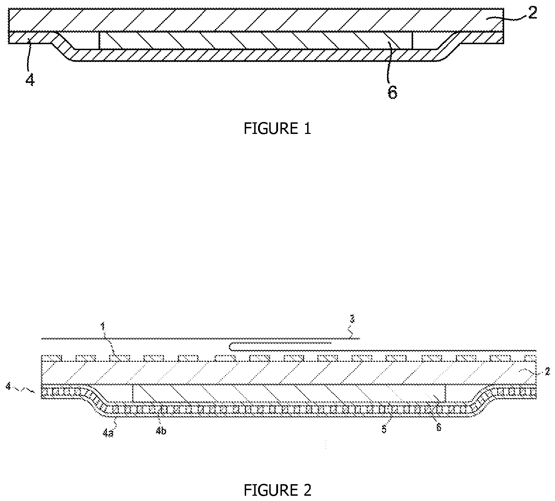

De tels pansements sont par exemple décrits par des vues schématiques dans les figures 1 et 2. Dans ces figures, le pansement est représenté en coupe droite.

Sur la figure 1, un support (4) est fixé à un matériau absorbant (2) et un non-tissé superabsorbant hydrodélitable (6) est intercalé entre ledit support (4) et le matériau absorbant (2).

Sur la figure 2, un matériau absorbant (2) est recouvert, du côté faisant face à la plaie, d'une couche d'interface discontinue (1) protégée par un protecteur pelable (3). Le support 4 est constitué par l'assemblage d'un film imperméable aux liquides et perméable à la vapeur d'eau (4a), et d'une armature ajourée enduite d'un gel de silicone adhésif (4b) sans obturer les ouvertures de l'armature. Le support (4) est fixé au matériau absorbant (2) par l'intercalage d'un voile non absorbant (5). Un non-tissé superabsorbant hydrodélitable (6) est intercalé entre le voile non absorbant (5) et le matériau absorbant (2). Le voile non absorbant (5) est fixé sur sa périphérie au matériau absorbant (2) de manière à emprisonner le non-tissé superabsorbant hydrodélitable (6) dont la dimension est inférieure à celle du matériau absorbant. Dans la figure 2, le support (4) a la même taille que le matériau absorbant (2).

### Support

Le support (4) est de préférence imperméable aux fluides et aux micro-organismes pathogènes extérieurs tout en assurant une perméabilité à la vapeur d'eau, de manière à éviter à la fois le contact de la plaie avec des liquides extérieurs et des bactéries et la macération de la plaie. On parle alors de « support imperméable et respirant ».

Le support faisant partie du pansement selon l'invention est constitué, selon un mode de réalisation préféré, d'un film continu. Le film est continu en ce sens qu'il n'a pas subi une étape de perforation.

Parmi les films utilisables, on peut citer à titre d'exemple les films en polyétheruréthane, en polyétheramide, ou en polyétherester. Le film peut en outre être remplacé par un complexe mousse/film ou un complexe textile/film.

L'épaisseur du film est par exemple comprise entre 5 et 200 microns, de préférence entre 10 et 75 microns, de préférence encore entre 10 et 50 microns.

Le film continu est imperméable aux liquides et perméable à la vapeur d'eau tels que ceux utilisés de façon courante pour la fabrication des pansements absorbants. Le film présente avantageusement un taux de transmission de vapeur d'eau (MVTR : Moisture Vapor Transmission Rate) supérieur à 1 000 g/m²/24 heures, de préférence supérieur ou égal à 7 000 g/m²/24 heures, de préférence encore supérieur ou égal à 10 000 g/m²/24 heures.

Le support imperméable et respirant peut se voir appliquer sur une de ses faces, de préférence sur la face située en regard de la plaie, un adhésif choisi parmi la liste des adhésifs acryliques, des adhésifs à base d'élastomères de silicone ou de polyuréthane, tels que les gels de silicone ou de polyuréthane, les adhésifs comprenant des hydrocolloïdes, ou les adhésifs à base d'élastomères thermoplastiques.

Selon un mode de réalisation alternatif de l'invention, le support faisant partie du pansement selon l'invention est constitué d'un film continu (4a) et d'une armature ajourée enduite d'un gel de silicone adhésif (4b) sans obturer les ouvertures de l'armature, ladite armature recouvrant l'intégralité de la surface du film. Cette armature ainsi enduite est avantageusement choisie de manière à ce que la valeur du taux de transmission de vapeur d'eau du support reste satisfaisante, notamment supérieure ou égale à 1000 g/m²/24 heures, de préférence supérieure ou égal à 3 000 g/m²/24 heures. Une technique de mesure du taux de transmission de vapeur d'eau en contact liquide est décrite dans la norme NF-EN 13726-2 (Chapitre 3.3).

L'armature pourra être constituée de tout matériau ajouré tels un film perforé, un filet thermoplastique, un textile comme par exemple un tissé, un tricot, ou un non tissé, de préférence élastique pour une meilleure tenue du pansement sur la peau. Un film perforé sera par exemple en polyéthylène ou en polypropylène. Un textile tissé sera par exemple en polyéthylène téréphtalate ou en polyamide. Le grammage de l'armature est de préférence compris entre 10 et 500 g/m², par exemple entre 20 et 300 g/m². L'armature peut être enduite de gel de silicone sur au moins une de ses faces, sur ses deux faces, voir sur toute sa surface. La taille des ouvertures de l'armature peut être comprise entre 0,1 et 5 mm, par exemple entre 0,5 et 3 mm. La surface ouverte de l'armature représente de préférence de 1 à 99%, de préférence de 25 à 90%, de préférence encore de 30 à 80%, de la surface du film continu, et la surface ouverte de l'armature une fois recouverte de gel de silicone représente de préférence de 10 à 99%, de préférence de 10 à 60%, de préférence encore de 25 à 75% de la surface du film continu.

Selon un mode de mise en œuvre, on utilisera un tricot ajouré, de préférence un tricot enduit de gel de silicone sur au moins une de ses surfaces sans obturer les ouvertures du tricot. L'ensemble de la masse du tricot est enrobée de gel de silicone, par exemple par trempage du tricot dans un mélange précurseur du gel de silicone.

Selon un autre mode de réalisation, l'armature est un film perforé enduit de gel de silicone sur au moins une de ses surfaces, de préférence sur une seule de ses faces, sans obturer les perforations du film, par exemple un film de polyuréthane perforé, qui pourra être fixé au film continu par la chaleur, les ultrasons, la haute fréquence ou un adhésif.

Le gel de silicone adhésif est un composé de silicone dont la structure est réticulée. Le gel de silicone présente une cohésion telle qu'il ne laisse pas de résidus sur la peau et reste accroché à l'armature quand on retire le pansement. Il peut être fabriqué à partir de précurseurs de silicone qui réticulent après leur mise en contact suivant une réaction d'hydrosilylation ou de condensation. De tels systèmes sont connus de l'art antérieur, par exemple dans les documents EP-A-0 251 810, EP-A-0 300 620 ou US-A-4 921 704. Les mélanges de précurseurs décrits dans ces documents comprennent pour l'essentiel :
- un composant A qui comprend au moins une polydiméthylsiloxane substituée par un groupe vinyle à chacune de ses extrémités, et un catalyseur de platine, et
- un composant B de polydiméthylsiloxane qui comprend au moins deux groupes hydrogénosilanes.

La mise en présence des deux composants provoque une réaction de réticulation (crosslinking reaction) des deux polydiméthylsiloxanes fonctionnalisées qui se produit avantageusement à température ambiante et peut être accélérée par la chaleur.

Des additifs comme des pigments, des inhibiteurs ou des charges de remplissage peuvent être incorporés à l'un au moins des deux composants.

Les précurseurs du gel de silicone adhésif peuvent être choisis parmi les produits suivants : Silbione RT Gel® 4712 A&B et Silbione RT Gel® 4717 A&B de Bluestar Silicones, Wacker Silgel® 612 de Wacker-Chemie GmbH, Nusil® MED-6340, Nusil® MED-6345, Nusil® MED3-6300, ou Nusil® MED12-6300 de Nusil Technology, et D-7-9800® de Dow Corning.

Le gel de silicone est de préférence choisi de telle façon que le support présente un pouvoir adhésif sur peau, selon la méthode EN 1939, supérieur à 40 cN/cm, et préférence 45 cN/cm. Un échantillon de support de 20 mm de large et 150 mm de long est posé sur l'avant-bras. Au bout de 10 minutes, on mesure le pouvoir adhésif avec un dynamomètre à une vitesse de traction de 900 mm/min avec un angle de 90°.

Le gel de silicone est de préférence appliqué sur l'armature ajourée sans obturer les ouvertures de l'armature à raison d'un grammage compris entre 100 et 500 g/m², de préférence entre 150 et 250 g/m², de manière à assurer un compromis entre un taux de transmission de vapeur d'eau suffisant et une adhésion au film continu ou à la peau suffisante.

A titre d'exemple, on peut utiliser un support constitué de l'association d'un film en polyuréthane de 30 µm d'épaisseur qui présente un taux de transmission de vapeur d'eau de l'ordre de 7000 g/m²/24 heures, et d'un tricot de polyester 40 g/m² enduit d'un gel de silicone sur toute sa surface à raison de 200 g/m². Ce support présente une épaisseur de l'ordre de 400 µm et une MVTR de l'ordre de 5000 g/m²/24 heures.

Dans un mode mise en œuvre, la taille du support est supérieure à celle du matériau absorbant et le matériau absorbant est centré sur le support de manière à créer des bordures adhésives.

Selon ce mode de réalisation alternatif, le support, constitué d'un film continu 4a et d'une armature ajourée enduite d'un gel de silicone adhésif 4b sans obturer les ouvertures de l'armature, est de préférence mince et souple, de manière à mieux épouser la forme du corps et suivre les mouvements sans risquer de se détacher. Le support est avantageusement conformable. Son épaisseur peut alors être comprise entre 100 et 1000 µm, de préférence entre 250 et 800 µm.

### Voile non absorbant

Selon un mode de réalisation alternatif de l'invention, le pansement peut comprendre un voile non absorbant (5) intercalé entre le matériau absorbant (2) et le support (4), et destiné à les assembler. Ce moyen de fixation est nécessaire au moins quand la surface du support est enduite d'un gel de silicone adhésif, et n'adhère pas de façon suffisante au matériau absorbant, en particulier en milieu humide.

Le voile intercalé entre le matériau absorbant et le support est un non tissé non absorbant de faible grammage. Le non tissé peut être tout type de non tissé couramment utilisé dans le domaine de l'hygiène et des pansements, notamment un non tissé filé lié (spun laid), cardé (carded) ou hydrolié (spun lace). Son grammage est de préférence compris entre 5 et 100 g/m².

Le voile est non absorbant en ce sens qu'il ne contient pas de fibres absorbantes telles que la rayonne, la viscose, les dérivés de cellulose, et qu'il ne contient pas de particules absorbantes.

Il pourra comprendre des fibres de polyamide, de polyester, de polyuréthane et ou de polyoléfines. Selon un mode de réalisation, le voile comprend des fibres de polyéthylène. Les fibres peuvent être monocomposants, ou bicomposants de type cœur/écorce ou côte-à-côte. On choisira par exemple un non tissé spun laid, de préférence de type spunbond.

Le voile non absorbant sera de préférence constitué de fibres hydrophobes, mais il pourra aussi être constitué de fibres hydrophiles et avoir subi un traitement pour le rendre hydrophobe. Le voile peut être constitué de plusieurs couches, dans la mesure où sa porosité est suffisante, la couche venant au contact du gel de silicone adhésif étant non absorbante et de préférence hydrophobe.

Le voile non absorbant est fixé au matériau absorbant sur toute sa surface, ou de préférence seulement sur sa périphérie par les technologies classiques de fixation telles la chaleur, les ultrasons, la haute fréquence, ou avec des adhésifs.

Dans le cadre de la présente invention, on utilisera par exemple un non tissé spun bond constitué de fibres de polyéthylène présentant un grammage compris entre 30 et 40 g/m², tel que celui commercialisé par la société Freudenberg sous la dénomination Berotex-PE 35g/m2 par la société Fiberweb.

### Matériau absorbant

Dans le cadre de la présente invention, le matériau absorbant (2) peut de préférence être choisi parmi une mousse ou un non-tissé.

La mousse absorbante peut être choisie parmi les mousses absorbantes hydrophiles et les mousses absorbantes hydrophobes rendues absorbantes par l'incorporation de particules absorbantes.

Des mousses absorbantes hydrophiles et leur procédé de fabrication, bien connus de l'homme de l'art, sont décrits dans les demandes de brevet WO 96/16099, WO 94/29361, WO 93/04101, EP 420 515, EP 392 788, EP 299 122, et WO 2004/074343. De telles mousses correspondent par exemple à la référence MCF03 commercialisée par la société AMS, ou à la référence 562-B commercialisée par la société RYNEL.

Selon une version préférée de la présente invention, on utilisera une mousse de polyuréthane hydrophile dont l'épaisseur est choisie en fonction de la capacité d'absorption souhaitée. Dans le cadre de la présente invention, on préférera tout particulièrement une mousse de polyuréthane de 1,5 à 5 mm d'épaisseur.

Le matériau absorbant (2) peut alternativement être un non-tissé.

Le non tissé peut être tout type de non tissé couramment utilisé dans le domaine de l'hygiène et des pansements, notamment un non tissé filé lié (spun laid) ou cardé (carded). Sa structure pourra être renforcée par aiguilletage ou par ajout d'un polymère thermoliant sous forme de poudre ou de fibres. Son grammage est de préférence compris entre 15 et 200 g/m².

Il peut être constitué de fibres thermoplastiques choisies parmi les polyoléfines, les polyamides, les polyesters, les polyuréthanes et ou de polyoléfines. Il peut comprendre des fibres absorbantes telles que la rayonne, la viscose et les dérivés de cellulose.

Il peut être constitué de fibres hydrophiles ou des fibres hydrophobes ayant subi un traitement hydrophile.

Il peut également comprendre des fibres superabsorbantes.

Lorsqu'il comprend des fibres superabsorbantes, le matériau absorbant est de préférence non hydrosoluble, lesdites fibres peuvent être monocomposants, ou bicomposants de type cœur/écorce ou côte-à-côte.

Selon un mode préféré de réalisation, le non tissé comprend des fibres superabsorbantes bicomposant de type cœur/écorce, ledit cœur étant en polyacrylonitrile et l'écorce étant en polyacrylate.

Selon une variante de l'invention, ledit non tissé peut être constitué d'un mélange de fibres superabsorbantes, bicomposant de type cœur/écorce, ledit cœur étant en polyacrylonitrile et l'écorce étant en polyacrylate , et de fibres non absorbantes thermoliantes, l'ensemble des fibres étant de préférence thermolié.

Par l'expression « superabsorbantes », on entend désigner ici des fibres qui présentent une très grande capacité d'absorption des liquides, de préférence supérieure ou égale à 20 g d'eau (ou de solution saline telle que du sérum physiologique) par gramme, et de préférence encore supérieure à 30 g d'eau par gramme.

Selon l'invention, les fibres superabsorbantes sont constituées de deux matériaux différents. Ces matériaux peuvent être répartis selon une configuration côte-à-côte, ou de préférence selon une configuration cœur-écorce.

Le premier matériau destiné à former une partie externe de la fibre, de préférence l'écorce, doit être apte à former un gel avec les exsudats de la plaie et sera avantageusement formé d'un ou plusieurs polymères réticulés et/ou partiellement réticulés. Ce premier matériau est formé de polyacrylate.

Le second composant qui formera de préférence le cœur des fibres superabsorbantes, sera de préférence non gélifiant et compatible avec le premier matériau pour garantir la stabilité de la fibre après formation d'un gel par le premier matériau. Il peut être formé de tout type de polymère stable en milieu aqueux et compatible avec le matériau de l'écorce pour conduire à une fibre bicomposante stable.

Ce second matériau est de préférence formé de polyacrylonitrile.

Les fibres superabsorbantes ont avantageusement un décitex compris entre 2 et 6 dtex.

Des fibres superabsorbantes pouvant être utilisées dans le non tissé absorbant selon l'invention sont par exemple commercialisées par la société TOYOBO CO LTD sous la dénomination LANSEAL® F.

Les fibres non absorbantes sont des fibres thermoliantes aptes à renforcer et stabiliser la structure tridimensionnelle du non tissé en formant une armature résultant de la liaison de ces fibres entre elles et/ou de ces fibres avec les fibres superabsorbantes.

Ces deuxièmes fibres peuvent être constituées d'une matière thermoplastique unique comme par exemple un polyéthylène, un polypropylène ou un polyester de bas point de fusion.

Avantageusement, ces deuxièmes fibres seront également constituées de deux matériaux différents répartis selon une configuration de type côte-à-côte ou de préférence cœur-écorce.

La longueur de ces fibres peut être de l'ordre de 10 à 100 mm, de préférence de 25 à 75 mm.

Dans le cadre de la présente invention, des fibres non absorbantes thermoliantes bicomposants de type cœur-écorce dans lesquelles le cœur est formé d'un polyester comme en particulier le polyéthylène téréphtalate, et l'écorce est formée de polyéthylène, sont particulièrement préférées.

Le ratio massique entre les fibres superabsorbantes et les fibres non absorbantes thermoliantes peut être compris entre 20/80 et 80/20, de préférence entre 60/40 et 80/20.

D'une façon générale, le non tissé absorant selon l'invention peut être obtenu à partir de mélanges incorporant plus de 50 % en poids, de préférence plus de 60% en poids, de fibres superabsorbantes.

D'excellents résultats ont été obtenus à l'aide d'un mélange comprenant 30 % en poids de fibres non absorbantes et 70 % en poids de fibres superabsorbantes.

Ce non tissé constituant le matériau absorbant du pansement selon l'invention peut notamment être obtenu par thermoliage, ou par aiguilletage et thermoliage du mélange de fibres.

L'opération de thermoliage permet d'améliorer la résistance au déchirement du non tissé après absorption, en créant des points d'ancrage entre les fibres du non tissé.

L'assemblage des fibres sera réalisé dans des conditions permettant d'obtenir un non tissé présentant une épaisseur comprise entre 0,3 et 3 mm, de préférence de 2 mm, et un grammage compris entre 30 et 400 g/m², de préférence de l'ordre de 100 g/m².

Le matériau non tissé constituant le matériau absorbant du pansement selon l'invention peut être fabriqué selon le procédé décrit dans le document GB 2401879.

### Non-tissé superabsorbant hydrodélitable

Le pansement selon l'invention comprend en outre un non-tissé superabsorbant hydrodélitable (6) placé entre le matériau absorbant (2) et le support de protection (4), ledit non-tissé superabsorbant hydrodélitable étant constitué de deux voiles hydrosolubles entre lesquels sont incorporées des particules ou granules de polymères super-absorbants.

Comme déjà défini, par « superabsorbant », on entend désigner ici des matériaux qui présentent une très grande capacité d'absorption des liquides, de préférence supérieure ou égale à 20 g d'eau (ou de solution saline telle que du sérum physiologique) par gramme, et de préférence encore supérieure à 30 g d'eau par gramme.

On entend par matériau « hydrophile », tout matériau ou composé présentant une affinité pour l'eau (ou une solution aqueuse), en particulier capable d'absorber l'eau sans toutefois s'y dissoudre ni s'y décomposer.

On entend par matériau « hydrosoluble », tout matériau ou composé capable de se dissoudre dans de l'eau (ou dans une solution aqueuse) afin de former une solution homogène.

On entend par matériau « hydrodélitable », tout matériau ou composé dont la structure se décompose en présence d'eau (ou d'une solution aqueuse), de sorte à former un milieu multi-phasique.

Le non-tissé superabsorbant hydrodélitable (6) a de préférence des dimensions inférieures à celles du matériau absorbant afin de pouvoir être emprisonné dans une enveloppe constituée du support de protection et du matériau absorbant dont les bords sont fixés entre eux. On dit, dans ce cas de figure, que le support de protection est fixé directement au matériau absorbant.

Selon un mode de réalisation alternatif, le pansement de l'invention comprend un voile non absorbant (5) intercalé entre le matériau absorbant (2) et le support (4), ledit voile permettant de les assembler. Ce moyen de fixation est nécessaire quand la surface du support enduite d'un gel de silicone adhésif n'adhère pas de façon suffisante au matériau absorbant, en particulier en milieu humide. On dit, dans ce cas de figure, que le support de protection est fixé indirectement au matériau absorbant.

Le non-tissé superabsorbant hydrodélitable est constitué de deux voiles hydrosolubles entre lesquels sont incorporés des particules ou granules (agrégats de particules) de polymères super-absorbants.

Les voiles hydrosolubles sont constitués par des matériaux choisis parmi la liste non limitative suivante :
- l'alcool polyvinylique (PVA),
- la poly(vinylpyrrolidone) (PVP),
- la carboxymethylcellulose (CMC),
- le polyéthylèneimine (PEI),
- les oxydes de polyéthylène (PEO),
- les polyacrylates,
- les alginates,
ou leurs mélanges et sels.

De préférence, les voiles hydrosolubles sont constitués par de l'alcool polyvinylique.

Selon un mode de mise en œuvre de la présente invention, le non tissé superabsorbant hydrodélitable peut incorporer des particules, qui peuvent être également des fibres ou des granules, de polymères superabsorbants dans une proportion comprise entre 1 et 95% en poids, de préférence de 40 à 90% en poids, du poids total du non tissé. Le polymère super-absorbant peut être choisi parmi les polymères acryliques dont leurs sels tels que les polyacrylates de sodium.

Selon un mode préféré de la présente invention on utilisera un non tissé comprenant entre chacun des deux voiles hydrosolubles fv des particules de polymères superabsorbants et des fibres de cellulose sans l'incorporation de matériaux thermoliants ou de latex. Selon une autre variante de la présente invention on pourra aussi employer comme non tissé superabsorbant hydrodélitable un non tissé constitué de deux voiles d'alcool polyvinylique entre lesquels sont incorporés des particules de polymères super-absorbants seules ou en association avec des liants, des adhésifs, des charges, des plastifiants, des solvants ou encore des fibres.

Dans le cadre de la présente invention, on préfère utiliser un voile d'alcool polyvinylique présentant une épaisseur comprise entre 0,05 et 1,5 mm, et/ou un grammage compris entre 10 et 200 g/m².

Dans le cadre de la présente invention, le non tissé superabsorbant hydrodélitable présente une épaisseur comprise entre 0,5 et 5 mm, et/ou un grammage compris entre 100 et 500 g/m².

### Couche d'interface

On pourra avantageusement déposer sur la surface du matériau absorbant, faisant face à la plaie une couche d'interface discontinue (1) destinée à entrer en contact avec la plaie. La couche d'interface est discontinue pour permettre l'accès des exsudats au matériau absorbant ; elle n'altère pas la plaie lors du retrait du pansement.

Selon un mode de réalisation de l'invention, le pansement ne comprend pas de trottoir et la couche d'interface est micro-adhérente à la plaie (comme illustré à la figure 1), c'est-à-dire qu'elle permet de fixer provisoirement le pansement sur la plaie, et celui-ci peut être retiré sans que la structure de la plaie ou de la peau périlésionnelle soit altérée, si bien que ce pansement est repositionnable et facilite les soins infirmiers. Cette fixation peut aider le personnel soignant ou l'utilisateur à recouvrir le pansement d'un moyen de contention, d'un ruban adhésif, d'une bande ou d'un filet de maintien.

La composition de la couche d'interface peut être hydrophobe, hydrophile ou amphiphile.

On peut ainsi citer des compositions à base de polymères silicone en particulier des gels de silicone, des gels de polyuréthane, des compositions à base d'élastomère incluant des hydrocolloïdes, voire des hydrogels comme par exemple des hydrogels à base de poly(AMPS).

Dans le cadre de la présente invention, on préférera tout particulièrement utiliser une couche discontinue de composition contenant un élastomère, un plastifiant et des hydrocolloïdes. Cette couche d'interface favorise le processus de cicatrisation en maintenant un environnement humide au niveau de la plaie, et permet également de véhiculer des actifs, ce qui n'est pas le cas des interfaces siliconées.

L'élastomère peut être choisi parmi les polymères séquencés poly(styrène-oléfine-styrène) triblocs éventuellement associés à des copolymères diblocs. Les copolymères triblocs peuvent être des copolymères séquencés poly(styrène-éthylène-butylène-styrène) (en abrégé SEBS) vendus sous la dénomination KRATON® G1651, KRATON® G1654 ou KRATON® G1652, ou des copolymères séquencés poly(styrène-éthylène-propylène-styrène) (en abrégé SEPS).

Parmi les composés plastifiants susceptibles d'être utilisés, on peut citer en particulier les huiles minérales, les polybutènes ou encore des dérivés de phtalate. D'une façon particulièrement préférée, on utilisera une huile plastifiante minérale choisie parmi les produits commercialisés sous les dénominations ONDINA®933 et ONDINA®919.

Comme hydrocolloïdes appropriés, on peut citer par exemple la pectine, les alginates, les gommes végétales naturelles comme en particulier la gomme de Karaya, les dérivés de cellulose tels que les carboxyméthylcelluloses et leurs sels de métal alcalin tels que le sodium ou le calcium connus sous la référence CMC Blanose® 7H4XF, ainsi que les polymères synthétiques à base de sels de l'acide acrylique superabsorbants, comme par exemple les produits commercialisés par la société BASF sous la dénomination LUQUASORB® 1003 ou par la société CIBA Specialty Chemicals sous la dénomination SALCARE® SC91 ainsi que les mélanges de ces composés.

La composition à base d'élastomère incluant des hydrocolloïdes peut inclure si nécessaire un ou plusieurs antioxydants, ainsi que le tensioactif MONTANOX® 80 ou le polymère SEPINOV® EMT 10 tous les deux commercialisés par la société SEPPIC pour optimiser la vitesse de gélification, la mouillabilité ou le relargage d'actifs éventuellement présents dans la composition.

Si l'on souhaite que la couche d'interface soit micro-adhérente ou adhérente, la composition à base d'élastomère incluant des hydrocolloïdes contient un produit tackifiant qui peut être choisi parmi les résines tackifiantes, les polyisobutylènes de bas poids moléculaire ou leurs mélanges. De façon générale, on préférera l'utilisation de résines hydrogénées telles que les résines ESCOREZ® de la série 5000 et tout particulièrement la résine ESCOREZ® 5380.

La composition peut contenir des principes actifs ayant un rôle favorable dans le traitement de la plaie. Parmi les substances susceptibles d'être utilisées dans le cadre de la présente invention on peut, à titre d'exemples, citer :
- les antibactériens comme par exemple les dérivés d'argent tels les sels d'argent ou d'autres métaux (par exemple le sulfate, le chlorure ou le nitrate d'argent et la sulfadiazine argentique), les complexes d'argent ou d'autres métaux (par exemple les zéolithes argent tel l'alphasan, ou les céramiques), le métrodinazole, la néomycine, le Polymyxine B, les pénicillines (Amoxycilline), l'acide clavulanique, les tétracyclines, la Minocycline, la chlorotétracycline, les aminoglycosides, l'Amikacine , la Gentamicine ou les probiotiques;
- les antiseptiques tels la chlorhexidine, le triclosan, le biguanide, l'hexamidine, le thymol, le Lugol, la Povidone iodée, le Chlorure de Benzalkonium et de Benzethonium;
- les anti-douleurs tels que le Paracétamol, la Codéine, le Dextropropoxyphène, le Tramadol, la Morphine et ses dérivés, les Corticoïdes et dérivés;
- les anti-inflammatoires tels que les Glucocorticoïdes, les anti-inflammatoires non stéroïdiens, l'Aspirine, l'Ibuprofène, le Kétoprofène, le Flurbiprofène, le Diclofénac, l'Acéclofénac, le Kétorolac, le Méloxicam, le Piroxicam, le Ténoxicam, le Naproxène, l'Indométacine, le Naproxcinod, le Nimésulide, le Célécoxib, l'Etoricoxib, le Parécoxib, le Rofécoxib, le Valdécoxib, la Phénylbutazone, l'acide niflumique, l'acide méfénamique;
- les actifs favorisant la cicatrisation tels que le Rétinol, la Vitamine A, la Vitamine E, la N-acétyl-hydroxyproline, les extraits de *Centella Asiatica,* la papaïne, les huiles essentielles de thym, de niaouli, de romarin et de sauge, l'acide hyaluronique, les oligosaccharides polysulfatés et leurs sels (en particulier les oligosaccharides sulfatés synthétiques ayant 1 à 4 unités oses tels que le sel de potassium du sucrose octasulfaté ou le sel d'argent du sucrose octasulfaté), le sucralfate, l'Allantoïne, l'urée, la metformine, les enzymes (par exemple protéolitiques telles la streptokinase, la tripsine ou la collagénase), des peptides ou des inhibiteurs de protéases;
- les anesthésiques tels que la benzocaïne, la lidocaïne, la dibucaïne, le chlorhydrate de pramoxine, la bupivacaïne, la mepivacaïne, la prilocaïne, ou l'étidocaïne.

On préférera employer des compositions micro-adhérentes à base d'élastomère contenant des hydrocolloïdes qui, pour un total de 100 % en poids, comprennent:
0,05 à 1 % en poids d'antioxydant ;
10 à 60 % en poids de résine tackifiante ;
2 à 20 %, de préférence de 12 à 16 %, en poids de carboxyméthylcellulose de sodium ;
10 à 65 % en poids d'une huile minérale plastifiante ;
5 à 25 % en poids d'un polymère tribloc poly(styrène-éthylène-butylène-styrène) ou poly(styrène-éthylène-propylène-styrène) ; et
1 à 15 % en poids d'un copolymère constitué d'un sel de l'acide 2-méthyl-2-[(1-oxo-2-propényl)amino]-1-propane-sulfonique et de l'ester 2-hydroxyéthyle de l'acide propénoïque.

Une autre composition à base d'élastomère contenant des hydrocolloïdes pourra comprendre, pour un total de 100 % en poids:
0,05 à 1 % en poids d'antioxydant ;
2 à 20 %, de préférence de 12 à 16 %, en poids de carboxyméthylcellulose de sodium ;
20 à 65 % en poids d'une huile minérale plastifiante ; et
3 à 25 % en poids d'un polymère tribloc poly(styrène-éthylène-butylène-styrène) ou poly(styrène-éthylène-propylène-styrène).

### Protecteur pelable

Le matériau absorbant ou le matériau absorbant recouvert d'une couche d'interface pourra être protégé, au moins sur sa face destinée à venir en contact avec la plaie, d'une pellicule de protection qui pourra être retirée par pelage avant utilisation du pansement.

Le protecteur pelable (3) peut être constitué d'une ou plusieurs parties pelables avant usage. Ce protecteur recouvre toute la surface du matériau absorbant ou l'ensemble de la surface du support, dans le cas où le pansement comprend un trottoir adhésivé.

Ce protecteur pourra être tout matériau couramment utilisé comme protecteur par l'homme du métier dans le domaine des pansements. Il pourra se présenter sous forme de film par exemple un film de polyoléfine tels que le polyéthylène ou le polypropylène, un film de polyester mais également sous forme d'un papier. Ce film est avantageusement traité sur l'une au moins de ses faces par un composé siliconé comme un silane, un composé fluoré, ou un composé siliconé et fluoré.

Ce protecteur devra être adapté à la nature micro-adhérente de la couche d'interface. Ce protecteur devra également, le cas échéant, être adapté à la nature de gel de silicone adhésif utilisé pour le support s'il comprend un trottoir.

Dans le cadre de la présente invention, on préférera en particulier l'utilisation d'un protecteur en deux parties, comme indiqué sur les figures 1 et 2.

Le protecteur pelable présente de préférence une épaisseur comprise entre 10 et 100 µm, par exemple de l'ordre de 50 µm.

Le produit commercialisé sous la référence Silflu® M1R88001 par la société Siliconature peut avantageusement être utilisé comme tel.

Le pansement de l'invention est avantageusement indiqué pour le traitement de toutes les plaies exsudatives ou chroniques (escarre, ulcère, comme par exemple un ulcère du pied du diabétique) et aiguës (brûlure du deuxième degré, dermabrasion, plaie traumatique, plaie post-opératoire).

Dans le cadre de la présente invention, on préférera l'utilisation d'un pansement à coins arrondis pour éviter un décollement prématuré.

Le pansement de l'invention peut se présenter sous forme de pansements individuels de petite dimension ou de dimension plus importante. Les pansements seront conditionnés individuellement dans une enveloppe scellée assurant une conservation en milieu stérile.

### Procédé de fabrication

La présente invention a également pour objet un procédé de fabrication du pansement décrit précédemment qui consiste à associer le matériau absorbant (2) au non-tissé superabsorbant hydrodélitable (6), et à fixer ledit matériau absorbant (2) au support (4) de manière à intercaler le non-tissé superabsorbant hydrodélitable entre le matériau absorbant et le support.

Lorsqu'un voile non absorbant (5) est intercalé entre le matériau absorbant et le support, un procédé de fabrication alternatif du pansement consiste à :
- associer le matériau absorbant (2) au non-tissé superabsorbant hydrodélitable (6),
- fixer le matériau absorbant au voile non absorbant (5) de manière à intercaler le non-tissé superabsorbant hydrodélitable entre le matériau absorbant et le voile non absorbant, puis
- assembler l'ensemble au support en mettant en contact le voile non absorbant et le support.

Selon un mode de réalisation particulier de l'invention, un procédé de fabrication du pansement décrit précédemment consiste dans une première étape à réaliser le support par enduction de l'armature et assembler l'armature enduite au film continu.

Le gel de silicone sera enduit sur l'armature en utilisant une des techniques d'enduction couramment employée par l'homme de l'art.

Selon cette version particulière de la présente invention, le gel de silicone est enduit sur les deux faces de l'armature, qui est assemblée au film continu alors que la réticulation du gel n'est pas complète, de manière à assurer la cohésion entre le film et l'armature ajourée. Dans ce mode de mise en œuvre, aucun adhésif n'est nécessaire pour faire adhérer l'armature au film imperméable aux liquides et perméable à la vapeur d'eau.

Dans cette version, le support peut être fabriqué selon la succession d'étapes suivantes :
- l'armature est recouverte sur ses deux faces d'un mélange des précurseurs du gel de silicone,
- l'armature est assemblée au film par exemple par calandrage, et
- la réticulation du gel de silicone est provoquée ou accélérée une fois que l'armature et le film ont été assemblés, en plaçant par exemple le support dans un four.

L'armature est par exemple plongée dans le mélange des précurseurs du gel de silicone, puis essorée dans un poste de laminage entre deux rouleaux. Une soufflerie permet de reconstituer les ouvertures de l'armature pour enlever le surplus de gel de silicone.

Dans une autre version, le gel de silicone est enduit sur une des deux faces de l'armature, et l'autre face est fixée au film continu par un adhésif. Elle peut aussi être fixée par la chaleur, les ultra-sons ou la haute fréquence et dans ce cas, l'armature et/ou le film continu pourront être thermoplastiques, de manière à les thermosceller.

Le support pourra être protégé en recouvrant sa face adhésive par une couche ou pellicule de protection. Cette couche de protection pourra être par exemple du papier ou un film de polyester.

Dans une deuxième étape, le voile non absorbant est associé au matériau absorbant, ce dernier étant fixé préalablement au non tissé superabsorbant hydrodélitable. Une bobine de matériau absorbant, une bobine de non-tissé superabsorbant hydrodélitable et une bobine de voile non absorbant sont déroulées puis détendues, avant d'être superposées, le non-tissé superabsorbant hydrodélitable étant intercalé entre les couches de de matériau absorbant et de voile non absorbant.

L'invention est illustrée par l'exemple suivant.

### Exemple

### 1: Préparation de deux pansements absorbants

On a fabriqué deux pansements comprenant une couche d'interface micro-adhérente, un matériau absorbant, un non-tissé superabsorbant (hydrodélitable pour le pansement selon l'invention, non-hydrodélitable pour le pansement comparatif), un voile non absorbant de polyéthylène, un support enduit de gel de silicone adhésif et un protecteur pelable.

Les matériaux suivants sont utilisés :
- Le support est un tricot de polyester 40 g/m² enduit sur ses deux faces et sur toute sa surface d'un gel de silicone adhésif (200 g/m²), qui a été laminé sur un film de polyuréthane de 30 µm d'épaisseur. Ce support présente une épaisseur de l'ordre de 300 µm et une MVTR de l'ordre de 5000 g/m²/24 heures.
- Le voile non absorbant est par exemple un non tissé polyéthylène de 35 g/m² vendu sous la référence Berotex-PE 35 g/m2 par Fiberweb.
- Le matériau absorbant est un non tissé comprenant des fibres superabsorbantes non hydrosolubles bicomposant de type cœur/écorce, ledit cœur étant en polyacrylonitrile et l'écorce étant en polyacrylate, et des fibres non absorbantes thermoliantes.
- Le non tissé superabsorbant est inséré entre le voile non absorbant et ledit matériau absorbant. Le non-tissé hydrodélitable est composé de :
- deux voiles périphériques hydrosolubles en alcool polyvinylique enfermant une composition superabsorbante ;
- ladite composition superabsorbante (200 g/m²) contenant au moins des particules de polymères superabsorbant.

Le non tissé superabsorbant non hydrodélitable utilisé dans le pansement comparatif est composé de :
- deux voiles périphériques cellulosiques hydrophiles enfermant une composition superabsorbante ;
- ladite composition superabsorbante (200 g/m²) contenant au moins des particules de polymères superabsorbant.

### 2. détermination de la rigidité des pansements absorbants

On a déterminé la rigidité des pansements chargés d'exsudats par un essai de flexion circulaire en mesurant la force maximum pour faire passer ledit pansement chargé au travers d'un orifice circulaire.

La procédure mise en œuvre se réfère à la norme ASTM D4032-08 : « Standard test method for stiffness of fabric by the circular blend procédure ».

### Principe :

### Echantillonnage et conditionnement

- Eprouvette : n ≥ 4
- solution NaCl/CaCl₂ comprenant du NaCl (8.298 g +/-5%) et du Ca Cl₂ (0.368g +-/5%)] :
- conditionner les échantillons au moins 24 heures à 21 +/- 2°C et 60 +/-15%HR
- condition de test : 21 +/- 2°C et 60 +/- 15%HR

### Appareillage et/ou réactifs

- Machine de traction référencée sous la dénomination DY32 par la société MTS à vitesse d'allongement constante, et adaptée pour une utilisation en compression.
- Piston de diamètre 25,4 mm monté sur un capteur de force
- Pince de serrage annulaire d'un diamètre inférieur de 44.4763 mm.

### Mode opératoire

- Préparer une solution d'essai comprenant du NaCl (8.298 g +/-5%) et du CaCl₂ (0.368g +-/5%)]. Cette solution permet, d'une part, de simuler les conditions d'humidité retrouvées au sein d'une plaie et, d'autre part, de simuler la concentration saline des exsudats retrouvés au niveau d'une plaie.
- Tremper les pansements à tester dans la solution d'essai pendant 30 minutes à 37°C + 2°C. Après 30minutes, on retire les pansements et on les laisse égoutter pendant environ 30 secondes.
- Installer le plateau et le guide cylindrique sur le dynamomètre en réglant le guide cylindrique environ 10 cm au-dessus du plateau
- Entrer les informations nécessaires à l'essai (vitesse d'essai : 500 mm/min) dans le logiciel du dynamomètre.
- Placer l'échantillon au centre du plateau inférieur, face adhésive vers le plateau
- Lancer le test et enregistrer la courbe de force mesurée relative à l'enfoncement du piston.
- Une fois le test terminé et la mâchoire supérieure revenue à sa distance initiale, retirer l'échantillon et relancer l'essai pour les autres échantillons.

### Résultats

Le résultat recherché est exprimé par la force en Newton mesurée par le capteur à des extensions de 5, 10 et 15 mm. Ces valeurs sont extraites de la courbe fournie par le logiciel.

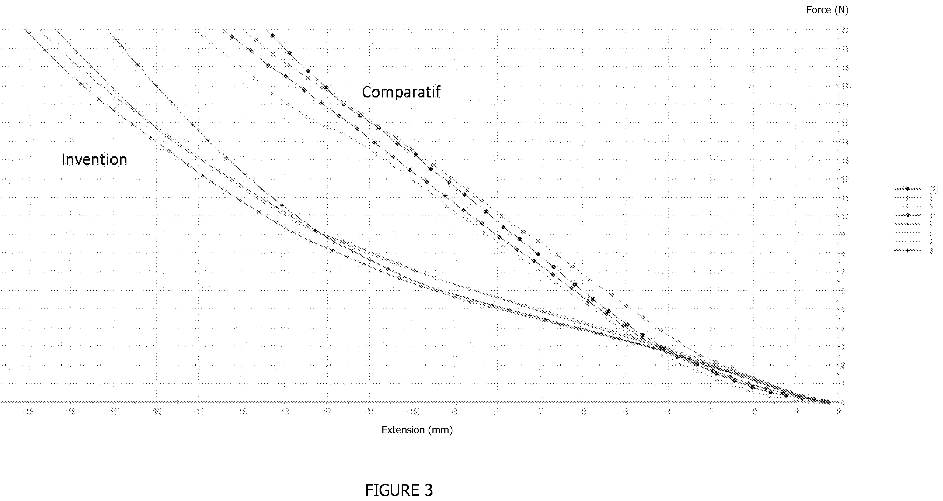

Pour chaque pansement, le test a été conduit 4 fois. Les résultats sont donnés dans les tableaux suivants et les courbes illustrant la force maximale en Newton sont présentées en Figure 3.

**Pansement comparatif :**

| | Extension (en mm) | | |
|---|---|---|---|
| Numéro de courbe | 5 | 10 | 15 |
| 1 | 4,16 N | 13,25 N | 23,9 N |
| 2 | 5,17 N | 13,59 N | 21,9 N |
| 3 | 3,76 N | 11,91N | 19,74 N |
| 4 | 4,12 N | 12,48 N | 21,14 N |

**Pansement Selon l'invention :**

| | Extension (en mm) | | |
|---|---|---|---|
| Numéro de courbe | 5 | 10 | 15 |
| 1 | 3,2 N | 6,23 N | 12,17 N |
| 2 | 3,5 N | 7,09 N | 12,98 N |
| 3 | 3,52 N | 6,96 N | 12,73 N |
| 4 | 3,43 N | 6,42 N | 14,07 N |

Il apparait clairement que la force maximum nécessaire pour passer le pansement au travers de l'orifice circulaire est moins élevée pour les pansements chargés selon l'invention par rapport aux pansements de l'art antérieur mettant un œuvre un non-tissé superabsorbant non hydrodélitable, ce qui se traduit par une plus grande flexibilité et donc une plus grande conformabilité des pansements selon l'invention.

Cette plus grande flexibilité et conformabilité est illustrée par les courbes de la figure 3.

## Revendications

1. Pansement comprenant :
- un matériau absorbant (2),
- un support de protection imperméable aux fluides et perméable à la vapeur d'eau (4), ledit support de protection étant fixé au dit matériau absorbant (2), ledit pansement comprenant en outre :
- un non-tissé superabsorbant hydrodélitable (6) placé entre le matériau absorbant (2) et le support de protection (4), ledit non-tissé superabsorbant hydrodélitable (6) étant constitué de deux voiles hydrosolubles entre lesquels sont incorporées des particules ou granules de polymères super-absorbants.

2. Pansement selon la revendication précédente, **caractérisé en ce que** le matériau absorbant (2) est choisi parmi une mousse ou un non-tissé.

3. Pansement selon la revendication 2, **caractérisé en ce que** la mousse absorbante est une mousse de polyuréthane hydrophile.

4. Pansement selon la revendication 2, **caractérisé en ce que** le non-tissé comprend des fibres superabsorbantes bicomposant de type cœur/écorce, ledit cœur étant en polyacrylonitrile et l'écorce étant en polyacrylate.

5. Pansement selon la revendication 4, **caractérisé en ce que** le non-tissé est formé d'un mélange desdites fibres superabsorbantes bicomposant et de fibres non absorbantes thermoliantes bicomposant de type cœur/écorce, ledit cœur étant de préférence en polyéthylène téréphtalate et l'écorce étant de préférence en polyéthylène.

6. Pansement selon l'une des revendications précédentes, **caractérisé en ce que** le support de protection imperméable aux fluides et perméable à la vapeur d'eau (4) est constitué d'un film continu (4a) et d'une armature ajourée enduite d'un gel de silicone adhésif (4b), sans obturer les ouvertures de l'armature.

7. Pansement selon l'une des revendications précédentes, **caractérisé en ce qu'**il comprend un voile non absorbant (5) intercalé entre le matériau absorbant (2) et le support (4), et destiné à les assembler.

8. Pansement selon la revendication précédente, **caractérisé en ce que** le voile non absorbant (5) présente un grammage compris entre 5 et 100 g/m².

9. Pansement selon l'une des revendications précédentes, **caractérisé en ce que** le matériau absorbant (2) est centré sur le support (4) dont la surface est supérieure, de manière à créer des bordures adhésives.

10. Pansement selon l'une des revendications 6 à 9, **caractérisé en ce que** le grammage du gel de silicone adhésif est compris entre 100 et 500 g/m².

11. Pansement selon l'une des revendications précédentes, **caractérisé en ce que** le matériau absorbant (2) est recouvert, sur la surface faisant face à la plaie, d'une couche d'interface discontinue (1) comprenant un élastomère, un plastifiant et des hydrocolloïdes.

12. Pansement selon l'une des revendications 6 à 11, **caractérisé en ce que** l'armature ajourée (4b) est un film ou un tricot perforé enduit de gel de silicone adhésif sur au moins une de ses surfaces, sans obturer les perforations du film.

13. Pansement selon l'une des revendications précédentes, **caractérisé en ce que** les voiles hydrosolubles sont constitués par des matériaux choisis parmi l'alcool polyvinylique, la poly(vinylpyrrolidone), la carboxymethylcellulose, le polyéthylèneimine, les oxydes de polyéthylène, les alginates, les polyacrylates, ou leurs mélanges et sels, de préférence l'alcool polyvinylique.

## Patentansprüche

1. Wundverband, umfassend:
- ein absorbierendes Material (2),
- einen flüssigkeitsundurchlässigen und wasserdampfdurchlässigen Schutzträger (4), wobei der Schutzträger an dem absorbierenden Material (2) befestigt ist, wobei der Wundverband ferner umfasst:
- einen wasser-zersetzbaren, superabsorbierenden Vliesstoff (6), der zwischen dem absorbierenden Material (2) und dem Schutzträger (4) positioniert ist, wobei der wasser-zersetzbare, superabsorbierende Vliesstoff (6) aus zwei wasserlöslichen Bahnen besteht, zwischen denen Partikel oder Körnchen aus superabsorbierenden Polymeren integriert sind.

2. Verband nach dem vorhergehenden Anspruch, **dadurch gekennzeichnet, dass** das absorbierende Material (2) aus einem Schaum oder einem Vliesstoff gewählt ist.

3. Verband nach Anspruch 2, **dadurch gekennzeichnet, dass** der absorbierende Schaum ein hydrophiler Polyurethanschaum ist.

4. Verband nach Anspruch 2, **dadurch gekennzeichnet, dass** der Vliesstoff superabsorbierende Bikomponentenfasern vom Typ Kern/Hülle umfasst, wobei der Kern aus Polyacrylnitril und die Hülle aus Polyacrylat besteht.

5. Verband nach Anspruch 4, **dadurch gekennzeichnet, dass** der Vliesstoff aus einer Mischung der superabsorbierenden Bikomponentenfasern mit thermisch bindenden, nicht absorbierenden Bikomponentenfasern vom Typ Kern-/Hülle gebildet ist, wobei der Kern bevorzugt aus Polyethylenterephthalat besteht und die Hülle bevorzugt aus Polyethylen besteht.

6. Verband nach einem der vorhergehenden Ansprüche, **dadurch gekennzeichnet, dass** der für Flüssigkeiten undurchlässige und für Wasserdampf durchlässige Schutzträger (4) aus einem durchgehenden Film (4a) und aus einer durchbrochenen Verstärkung besteht, die mit einem haftenden Silikongel (4b) beschichtet ist, ohne die Öffnungen der Verstärkung zu verschließen.

7. Verband nach einem der vorhergehenden Ansprüche, **dadurch gekennzeichnet, dass** er eine nicht absorbierende Bahn (5) umfasst, die zwischen dem absorbierenden Material (2) und dem Träger (4) angeordnet und dazu ausgebildet ist, diese zusammenzufügen.

8. Verband nach dem vorhergehenden Anspruch, **dadurch gekennzeichnet, dass** die nicht absorbierende Bahn (5) ein Flächengewicht zwischen 5 und 100 g/m² aufweist.

9. Verband nach einem der vorhergehenden Ansprüche, **dadurch gekennzeichnet, dass** das absorbierende Material (2) zentral angeordnet ist auf dem Träger (4), dessen Oberfläche oben liegt, so dass haftende Ränder gebildet werden.

10. Verband nach einem der Ansprüche 6 bis 9, **dadurch gekennzeichnet, dass** das Flächengewicht des haftenden Silikongels zwischen 100 und 500 g/m² liegt.

11. Verband nach einem der vorhergehenden Ansprüche, **dadurch gekennzeichnet, dass** das absorbierende Material (2) auf der der Wunde zugewandten Oberfläche mit einer unterbrochenen Grenzschicht (1) bedeckt ist, welche ein Elastomer, einen Weichmacher und Hydrokolloide umfasst.

12. Verband nach einem der Ansprüche 6 bis 11, **dadurch gekennzeichnet, dass** die durchbrochene Verstärkung (4b) ein perforierter Film oder Stoff ist, der auf mindestens einer seiner Oberflächen mit haftendem Silikongel beschichtet ist, ohne die Perforationen des Films zu verschließen.

13. Verband nach einem der vorhergehenden Ansprüche, **dadurch gekennzeichnet, dass** die wasserlöslichen Bahnen aus Materialien bestehen, gewählt aus Polyvinylalkohol, Poly(vinylpyrrolidon), Carboxymethylcellulose, Polyethylenimin, Polyethylenoxiden, Alginaten, Polyacrylaten oder Mischungen und Salzen davon, bevorzugt Polyvinylalkohol.

## Claims

1. Dressing comprising:
- an absorbent material (2),
- a fluid-impermeable and water-vapour-permeable protective substrate (4), said protective substrate being attached to said absorbent material (2), said dressing further comprising:
- a water-disintegrating superabsorbent nonwoven (6) placed between the absorbent material (2) and the protective substrate (4), said water-disintegrating superabsorbent nonwoven (6) consisting of two water-soluble webs between which superabsorbent polymer particles or granules are incorporated.

2. Dressing according to the preceding claim, **characterised in that** the absorbent material (2) is selected from a foam or a nonwoven.

3. Dressing according to claim 2, **characterised in that** the absorbent foam is a hydrophilic polyurethane foam.

4. Dressing according to claim 2, **characterised in that** the nonwoven comprises core/shell type bicomponent superabsorbent fibres, said core being made of polyacrylonitrile and the shell being made of polyacrylate.

5. Dressing according to claim 4, **characterised in that** the nonwoven is formed of a blend of said bicomponent superabsorbent fibres and of core/shell type bicomponent thermobinding non-absorbent fibres, said core being preferably made of polyethylene terephthalate and the shell being preferably made of polyethylene.

6. Dressing according to one of the preceding claims, **characterised in that** the fluid-impermeable and water-vapour-permeable protective substrate (4) consists of a continuous film (4a) and of an open-work reinforcement coated with an adhesive silicone gel (4b), without occluding the openings of the reinforcement.

7. Dressing according to one of the preceding claims, **characterised in that** it comprises a non-absorbent web (5) inserted between the absorbent material (2) and the substrate (4), and intended to assemble them.

8. Dressing according to the preceding claim, **characterised in that** the non-absorbent web (5) has a basis weight between 5 and 100 g/m².

9. Dressing according to one of the preceding claims, **characterised in that** the absorbent material (2) is centred on the substrate (4) the surface thereof is greater, so as to create adhesive borders.

10. Dressing according to one of claims 6 to 9, **characterised in that** the basis weight of the adhesive silicone gel is between 100 and 500 g/m².

11. Dressing according to one of the preceding claims, **characterised in that** the absorbent material (2) is covered, on the surface facing the wound, with a discontinuous interface layer (1) comprising an elastomer, a plasticiser and hydrocolloids.

12. Dressing according to one of claims 6 to 11, **characterised in that** the open-work reinforcement (4b) is a perforated film or knit fabric coated with adhesive silicone gel on at least one of the surfaces thereof, without occluding the film perforations.

13. Dressing according to one of the preceding claims, **characterised in that** the water-soluble webs consist of materials selected from polyvinyl alcohol, polyvinyl pyrrolidone, carboxymethylcellulose, polyethyleneimine, polyethylene oxides, alginates, polyacrylates, or mixtures and salts thereof, preferably polyvinyl alcohol.
